# FASCICULE DE BREVET EUROPEEN

(11) **EP 1 092 427 B1**
(45) Date de publication et mention de la délivrance du brevet: **23.05.2007**
(21) Numéro de dépôt: 99402424.8
(22) Date de dépôt: 04.10.1999
(51) Int. Cl.: A61K 8/64, A61K 38/06, A61Q 5/00, A61Q 19/00

(54) **Utilisation d'une peptide prévenant les réactions d'intolérance de la peau, notamment dans des compositions cosmétiques**
Verwendung eines Hautüberempfindlichkeit vermeidenden Peptids, insbesondere bei kosmetischen Zusammensetzungen
Use of a peptide avoiding skin intolerance reactions, especially in cosmetic compositions

(43) Date de publication de la demande: 18.04.2001
(73) Titulaire: L'ORÉAL, 75008 Paris (FR)
(72) Inventeur: Mahe, Yann, 91390 Morsang sur Orge (FR); Duranton, Albert, 78600 Maisons Laffitte (FR)
(74) Mandataire: Le Coupanec, Pascale A.M.P.

(56) Documents cités:
- EP-A- 0 764 444
- RHEINS ET AL.: "Alpha-melanocyte stimulating hormone modulates..." JOURNAL OF INVESTIGATIVE DERMATOLOGY, vol. 93, no. 4, octobre 1989 (1989-10), pages 511-517, XP002109102
- CODY ET AL.: "Cyclic melanotropins. 5. Importance of the C-Terminal Tripeptide (Lys-Pro-Val)" JOURNAL OF MEDICAL CHEMISTRY, vol. 27, no. 9, 1984, pages 1186-1190, XP000876818

## Description

L'invention concerne l'utilisation pour la préparation d'une composition cosmétique, comme additif, d'un peptide particulier permettant notamment de prévenir ou de réduire les réactions d'intolérance de la peau.

L'utilisation d'un tel peptide comme additif dans une composition cosmétique permet de diminuer les risques de développement de dermatites de contact (dites aussi eczémas de contact) ou d'autres réactions de la peau résultant d'une hypersensibilité de contact.

On sait que les réactions d'hypersensibilité de contact sont provoquées par des allergènes de faible poids moléculaire (haptènes) qui sont capables de se lier avec des protéines présentes dans les cellules épidermiques, y compris des protéines de cellules présentatrices d'antigène (cellules de Langherans), pour former des complexes (ou conjugués) protéine-haptène. Les cellules de présentation de l'antigène de l'épiderme migrent ensuite et sensibilisent des lymphocytes T. Après un temps de latence qui est d'environ 4 à 5 jours, lorsqu'un site de l'épiderme se trouve ensuite de nouveau au contact de l'allergène (réaction déclenchante), les cellules T sensibilisées migrent vers ce site et induisent la libération de cytokines qui provoquent l'arrivée de macrophages et de cellules T non sensibilisées qui sont à leur tour activées. Cet afflux de cellules peut se traduire notamment par la formation d'un oedème ou de vésicules survenant généralement au bout de quelques heures, voire 1 ou 2 jours après la réaction déclenchante, chez les personnes déjà sensibilisées.

Contrairement aux autres réactions allergiques, provoquées par une production excessive d'immunoglobulines IgE, les réactions d'hypersensibilité de contact sont liées principalement à une réponse immunologique cellulaire. L'hypersensibilité de contact est rattachée par les immunologistes à l'hypersensibilité retardée de type IV, encore appelée hypersensibilité à médiation cellulaire.

Les substances sensibilisantes peuvent être très diverses. Ce sont notamment des métaux ou des sels métalliques (chrome, nickel, cobalt, mercure), le formol, les résines synthétiques (résines époxy, résines acryliques et phénoliques), les latex, des médicaments d'application locale, certains cosmétiques, des colorants ou précurseurs de colorants (paraphénylènediamine, aniline, aminophénols), des pesticides, certains tissus synthétiques, certains produits végétaux (notamment pentadécylcatéchols, ou "poison ivy"), ou des agents conservateurs.

Ainsi, de nombreuses substances sont susceptibles de provoquer des dermatites de contact, aussi bien dans la vie professionnelle que dans la vie quotidienne.

L'hypersensibilité de contact, qui se manifeste notamment par une irritation peut, dans les formes aiguës, provoquer un érythème, un oedème, la formation de vésicules, ou encore un prurit ou une sensation de brûlure.

Les formes les plus graves des dermatites de contact nécessitent un traitement pharmaceutique par les corticostéroïdes. Ceux-ci sont donc utilisés postérieurement à un déclenchement de la réaction d'hypersensibilité. Leur utilisation thérapeutique *a posteriori*, bien qu'efficace, est donc curative et non préventive. En outre, leur mode d'action peut provoquer à long terme des effets indésirables (notamment blanchiment de la peau, atrophie de la peau et risque de surinfection cutanée).

Par ailleurs, on sait que de très nombreuses molécules, d'une grande diversité, sont susceptibles d'être utilisées comme constituants des compositions cosmétiques. On sait aussi qu'on observe actuellement une augmentation du nombre de cas de réactions croisées d'hypersensibilité de contact. Ainsi, l'application de compositions cosmétiques risque de provoquer de telles réactions chez les utilisateurs.

Il est donc souhaitable de pouvoir disposer de nouveaux agents permettant de prévenir les réactions d'hypersensibilité de contact ou de diminuer les risques de survenue d'une telle réaction chez les humains, et notamment chez les personnes ayant des peaux hypersensibles, dites aussi peaux intolérantes et/ou réactives.

EP 0 764 444 et WO 88/00833 décrivent l'utilisation de peptides ou dérivés peptidiques contenant la séquence Lys-Pro-Val, à titre d'agent de traitement ou de réduction de l'inflammation.

La présente invention repose sur la découverte des propriétés de certains peptides capables de prévenir les réactions d'hypersensibilité de contact ou de diminuer les risques de survenue d'une telle réaction. Un tel effet est clairement différent d'un effet anti-inflammatoire, comme le montre par exemple la partie expérimentale ci-après.

L'invention concerne plus précisément l'utilisation de peptides particuliers contenant la séquence peptidique Lysine-Proline-Valine comme additifs dans une composition cosmétique ou encore comme principes actifs dans la préparation d'un médicament destiné à supprimer ou diminuer les réactions d'hypersensibilité de contact, à l'exception de l'utilisation de la mélanotropine. La mélanotropine, encore appelée α-MSH est un peptide naturel connu ayant 13 résidus d'acides aminés (tous de configuration L) et contenant aux positions 11 à 13 la séquence peptidique indiquée.

Une activité de modulateur de la réponse à l'hypersensibilité de contact a été décrite, pour la mélanotropine, par RHEINS et al., J. Invest. Dermatol. 93, 511-517(1989).

US 4 874 744 décrit la mise en oeuvre de l'α-mélanocyte stimulating hormone (α-MSH) pour le traitement des phénomènes d'hypersensibilité ou d'allergie de contact.

Chacun des résidus d'acides aminés des peptides utilisés selon la présente invention peut être de configuration quelconque, L ou D.

L'invention a pour objet de prévenir ou de réduire les réactions d'intolérance de la peau liées à une hypersensibilité de contact, en appliquant sur la peau ou les phanères une composition cosmétique usuelle contenant comme additif au moins un peptide contenant la séquence peptidique Lysine-Proline-Valine, à l'exception de la mélanotropine, ou un dérivé d'un tel peptide.

On entend par "phanères" les cheveux, les ongles, et les poils (notamment cils et sourcils).

L'invention s'adresse notamment aux personnes ayant une peau hypersensible ou intolérante.

L'invention a également pour objet l'utilisation comme principe actif, dans la préparation d'un médicament destiné à lutter (de façon préventive ou curative) contre les réactions d'hypersensibilité de contact, d'au moins un peptide (ou d'un dérivé d'un tel peptide) contenant la séquence peptidique Lysine-Proline-Valine, tel que défini précédemment, à l'exception de l'utilisation de la mélanotropine.

Le peptide utilisé selon l'invention peut contenir notamment de 3 à 10 résidus d'acides aminés, et en particulier 3, 4, 5 ou 6 résidus d'acides aminés, qui peuvent être chacun indépendamment de configuration L ou D.

A titre d'exemple, au moins un résidu d'acide aminé du peptide, en particulier au moins un résidu d'acide aminé de la séquence peptidique Lysine-Proline-Valine, et notamment le résidu proline, peut avoir la configuration D. L'invention inclut l'utilisation de peptides tels que définis précédemment dans lesquels les résidus d'acides aminés constituant la séquence peptidique Lysine-Proline-Valine ont tous la configuration D. Les peptides utilisés selon l'invention sont notamment ceux dans lesquels, en outre, au moins une partie (y compris tous) des résidus d'acides aminés autres que ceux de la séquence Lys-Pro-Val ont la configuration D.

L'invention concerne notamment l'utilisation de peptides contenant au moins la séquence peptidique de formule 1 :

Lys-Pro-Val (I)

ainsi que l'utilisation de dérivés de ces peptides, à l'exception de l'utilisation de la mélanotropine. Les dérivés des peptides contenant la séquence de formule 1 sont par exemple ceux dont au moins un groupement fonctionnel (en particulier les groupements amines et carboxyliques) est protégé avec un groupement protecteur. Bien entendu, le groupement protecteur doit être compatible avec une utilisation dans le domaine des cosmétiques ou de la pharmacie. Les groupements protecteurs usuels sont bien connus. Les dérivés de peptides comprennent notamment ceux pour lesquels le groupement carboxylique terminal, et les autres groupements carboxyliques éventuellement présents, sont sous la forme d'un ester (par exemple ester d'alkyle inférieur) ou d'un amide, et/ou ceux pour lesquels le groupement amine N-terminal, et les autres groupements amines éventuellement présents, sont sous forme acylée (par exemple acétylée). Plus généralement, les dérivés des peptides contenant la séquence peptidique de la formule 1 incluent non seulement les sels d'addition avec des acides organiques carboxyliques, et non seulement les acétates, mais aussi d'autres sels d'addition tel que par exemple les trifluoroacétates, ainsi que les sels d'addition avec les acides minéraux tels que les sulfates, les chlorhydrates, etc. Les dérivés incluent également les sels résultant de la salification du groupe carboxylique (ou des groupes carboxyliques), et notamment les sels de métaux alcalins ou alcalino-terreux tels que les sels de sodium ou de calcium.

Parmi les peptides utilisables selon l'invention, on citera en particulier ceux dans lesquels le résidu Val, dans la séquence Lys-Pro-Val, constitue l'extrémité C-terminale de cette séquence, et notamment ceux pour lesquels la séquence Lys-Pro-Val constitue l'extrémité C-terminale du peptide (l'acide aminé C-terminal étant Val). On citera à titre d'exemples les peptides qui contiennent au moins l'une des séquences tripeptidiques suivantes : D-Lys-D-Pro-D-Val, D-Lys-D-Pro-L-Val, L-Lys-D-Pro-D-Val ou L-Lys-D-Pro-L-Val. Les tripeptides constitués par les séquences qui viennent d'être mentionnées sont appelés peptides de formule 1a, 1b, 1c et 1d, respectivement.

Le dérivé du peptide de formule 1a dont le résidu lysine est acétylé et dont le groupement carboxylique du résidu valine est amidifié est symbolisé par la formule II

Ac-D-Lys-D-Pro-D-Val-NH₂ (II)

Les peptides utilisés selon l'invention, et leurs dérivés, peuvent être préparés selon les techniques usuelles de synthèse peptidique.

Les compositions contenant le peptide utilisé selon l'invention peuvent être administrées par voie orale, parentérale ou topique.

A cet effet, elles peuvent être présentées notamment sous la forme de comprimés, de gélules, de solutions buvables, de solutions injectables, de lyophilisats pour solutions injectables, de lotions, de gels ou d'émulsions liquides ou semi-solides.

De telles compositions contiennent le peptide utilisé selon l'invention à des concentrations pouvant varier généralement de 10⁻¹² M à 10⁻² M, et en particulier de 10⁻⁷ M à 10⁻³ M.

Ces compositions sont préparées selon les méthodes usuelles.

Les compositions pharmaceutiques obtenues selon l'invention contiennent, outre le principe actif, éventuellement associé à d'autres principes actifs, au moins un véhicule pharmaceutique approprié.

L'invention concerne également l'utilisation d'au moins un peptide ou dérivé de peptide, tel que défini ci-dessus, dans des compositions cosmétiques (notamment compositions pour la peau, pour les ongles ou pour les cheveux), comme additif destiné à supprimer ou réduire les risques de réactions d'hypersensibilité de contact pour les utilisateurs de la composition. Il s'agit de diminuer aussi bien les risques d'une réaction à des agents extérieurs potentiellement sensibilisants que les risques d'une réaction aux substances présentes dans la composition cosmétique qui constituent des allergènes potentiels.

Les compositions cosmétiques ainsi obtenues contiennent, outre le peptide, les ingrédients et véhicules usuels présents dans le type de composition envisagé. Ces compositions sont notamment des lotions, crèmes ou gels pour le visage, le cou ou les mains, des rouges à lèvres, des compositions de maquillage, de démaquillage ou de nettoyage de la peau, des mascaras, des fonds de teint, des poudres pour le visage, des savons, des parfums, des vernis à ongles, ou encore des compositions capillaires comme par exemple des shampooings, des lotions capillaires, des compositions de teinture pour cheveux, des compositions de déformation permanente pour cheveux, etc.

L'intérêt de la présence d'un peptide utilisé selon l'invention dans des compositions pour cheveux résulte évidemment du fait que lors de l'utilisation, ces compositions sont le plus souvent en contact avec le cuir chevelu, d'où un risque d'allergie de contact. Dans le cas de compositions pour les ongles, l'intérêt est que souvent les ongles sont en contact avec la peau.

Dans la partie expérimentale ci-après, le sigle TNCB désigne le trinitrochlorobenzène.

### ETUDE PHARMACOLOGIQUE

### 1) Etude de l'effet du peptide de formule II sur la réponse à la sensibilisation par le TNCB, sur la souris

### a) Diminution de la réaction d'hypersensibilité de contact après sensibilisation

L'étude est effectuée sur des souris femelles Balb/C âgées de 7 à 10 semaines.

On injecte une dose de 1,5 µg du peptide étudié (en solution dans du tampon PBS contenant 0,1 % de sérum de souris) dans une veine de la queue des souris 2 heures avant la réaction de sensibilisation.

La réaction de sensibilisation consiste à appliquer au pinceau 100 µl d'une solution à 0,15 % de TNCB dans un mélange acétone-huile d'olive (4:1) sur la peau rasée de l'abdomen des souris.

La réaction déclenchante est réalisée 7 jours après la réaction de sensibilisation. Cette réaction déclenchante consiste à appliquer sur les deux faces d'une oreille 10 microlitres d'une solution à 0,8 % de TNCB dans un véhicule constitué par le mélange acétone-huile d'olive déjà mentionné ci-dessus.

Sur l'autre oreille, on applique, pour comparaison, uniquement ce mélange d'acétone et d'huile d'olive.

On évalue l'intensité d'une éventuelle réaction d'hypersensibilité de contact par estimation de l'importance de l'oedème de l'oreille traitée par le TNCB, en comparaison avec l'oreille traitée par le seul véhicule. Pour cela, on mesure et compare l'épaisseur des oreilles à l'aide d'une vis micrométrique. Cette mesure est effectuée 24 heures après la réaction déclenchante.

On applique également le TNCB sur l'oreille de souris témoins n'ayant pas été préalablement sensibilisée (témoins négatifs).

Les témoins positifs ne reçoivent pas le peptide avant la réaction de sensibilisation.

### Résultats

Dans cette expérience, l'augmentation de l'épaisseur de l'oreille chez les témoins positifs est en moyenne de 9,8×10⁻² mm. Chez les témoins négatifs, cette augmentation est de l'ordre de 1×10⁻² mm. Pour les animaux traités avec le peptide étudié, cette augmentation n'est que de 2,5×10⁻² mm environ.

Ces résultats montrent que le peptide étudié réduit fortement la réaction d'hypersensibilité de contact.

### b) Réduction de la réaction d'hypersensibilité de contact après une seconde sensibilisation

14 jours après la fin de l'expérience décrite en a) ci-dessus, on soumet les mêmes souris à une nouvelle réaction de sensibilisation sur la peau rasée du dos, comme décrit en a).

7 jours plus tard, on effectue le test de déclenchement par application du TNCB sur l'oreille, et 24 heures plus tard, on mesure le degré de gonflement de l'oreille.

Cette seconde expérience a pour but de faire une distinction entre une éventuelle immunosuppression temporaire aspécifique et une tolérance immunologique spécifique.

### Résultats

Pour les témoins positifs, l'augmentation moyenne de l'épaisseur de l'oreille est de l'ordre de 14×10⁻² mm. Pour les animaux qui avaient été traités par le peptide II dans la première expérience, tout comme pour les témoins négatifs, le degré de gonflement de l'oreille est de l'ordre de 3,5×10⁻² mm.

Ces résultats montrent que les animaux traités par le peptide étudié avant la première sensibilisation n'ont pas développé une hypersensibilité de contact après application d'une seconde dose sensibilisante de TNCB. Les souris ainsi traitées sont donc devenues tolérantes au TNCB.

### c) Effet du peptide de formule II en application topique

On opère comme en a) ci-dessus, mais le peptide, incorporé dans une crème de type eau-dans-l'huile, est appliqué sur la zone à sensibiliser 2 heures avant la sensibilisation.

24 heures après la réaction déclenchante, l'augmentation de l'épaisseur de l'oreille est en moyenne de 3×10⁻² mm chez les souris traitées et de 7×10⁻² mm chez les témoins positifs.

### 2) Etude de l'effet par voie topique chez l'homme

Cette étude a été effectuée sur des patients volontaires âgés de 18 à 65 ans, chez lesquels une allergie de contact au nickel était suspectée. Ces patients étaient exempts d'autres maladies cutanées, et les résultats de cette étude montrent qu'ils n'avaient pas d'allergie aux constituants de la crème utilisée comme véhicule.

Au jour 0, on applique avec un pansement occlusif, sur des zones de la peau du dos, d'une part une crème contenant 100 µM du peptide de formule II, et d'autre part la crème seule, sans peptide, utilisée comme placebo.

Deux heures plus tard, on applique sur les mêmes zones de la peau, et aussi sur une zone adjacente non prétraitée, des patchs imprégnés de vaseline contenant du sulfate de nickel à une concentration de 5 %.

24 heures plus tard, on examine les patients. Ceux qui ont développé une réaction d'hypersensibilité de contact sur la zone traitée par le placebo (et aussi sur la zone adjacente n'ayant reçu que l'application de sel de nickel), et qui sont donc des personnes qui étaient déjà sensibilisées au nickel, sont au nombre de 14. Sur ces 14 personnes, 7 n'ont développé aucune réaction d'hypersensibilité de contact sur la zone traitée par le peptide.

Ces résultats montrent que le peptide étudié agit localement, sur le site d'application, puisque la zone cutanée adjacente n'ayant reçu que l'application de sel de nickel a bien donné lieu à une réaction d'hypersensibilité de contact.

### EXEMPLES DE COMPOSITIONS

### EXEMPLE 1 : Lotion pour le cuir chevelu

- Peptide de formule II 12,5.10⁻⁶ g
- 2,4-diaminopyrimidine-3-oxyde 0,75 g
- Ethanol à 95% 30 g
- Parfum qs
- Colorants qs
- Eau déminéralisée qsp 100 g

### EXEMPLE 2 : Crème de soin pour la peau (émulsion huile-dans-l'eau)

- Mélange (80:20) d'alcool cétylstéarylique et d'alcool cétylstéarylique oxyéthyléné à 33 moles d'oxyéthylène 5 g
- Monostéarate de glycérol 1,5 g
- Alcool cétylique 0,75 g
- Huile de vaseline 10 g
- Polydiméthylsiloxane 0,75 g
- Glycérine 4 g
- Conservateurs qs
- Peptide de formule II 5 mg
- Eau déminéralisée qsp 100 g

### EXEMPLE 3 : Solution injectable par voie intradermique

- Peptide de formule II 0,7 mg
- Solution aqueuse stérile apyrogène à 9% de NaCl qsq 1 ml

## Revendications

1. Utilisation d'au moins un peptide, contenant de 3 à 10 résidus d'acides aminés et contenant la séquence peptidique Lysine-Proline-Valine, pour la préparation d'une composition cosmétique destinée à être administrée par voie topique, à titre d'additif pour prévenir, ou réduire, les risques de survenue des réactions d'intolérance de la peau liées à une hypersensibilité de contact, pour les utilisateurs de ladite composition.

2. Utilisation selon la revendication 1, dans laquelle le peptide utilisé contient de 3 à 6 résidus d'acides aminés.

3. Utilisation selon l'une quelconque des revendications précédentes, dans laquelle l'un au moins des résidus d'acides aminés, et en particulier le résidu proline, de ladite séquence peptidique a la configuration D.

4. Utilisation selon l'une quelconque des revendications précédentes, dans laquelle les résidus d'acides aminés constituant la séquence peptidique Lysine-Proline-Valine ont tous la configuration D.

5. Utilisation selon l'une quelconque des revendications précédentes, dans laquelle tous les résidus d'acides aminés du peptide ont la configuration D.

6. Utilisation selon l'une quelconque des revendications précédentes, dans laquelle ledit peptide est un peptide dont au moins un groupement fonctionnel est protégé avec un groupement protecteur, ou un de sels d'addition avec des acides organiques carboxyliques, des sels de métaux alcalins ou alcalino-terreux.

7. Utilisation selon l'une quelconque des revendications précédentes, dans laquelle ledit additif est le tripeptide D-Lys-D-Pro-D-Val

8. Utilisation selon l'une quelconque des revendications précédentes, dans laquelle ladite composition est choisie parmi des lotions, des crèmes ou des gels pour le visage, le cou ou les mains, des rouges à lèvres, des compositions de maquillage, de démaquillage ou de nettoyage de la peau, des mascaras, des fonds de teint, des poudres pour le visage, des savons, des parfums, des vernis à ongles, des shampoings, des lotions capillaires, des compositions de teinture pour cheveux et des compositions de déformation permanente des cheveux.

9. Utilisation comme principe actif, dans la préparation d'un médicament destiné à être administré par voie topique, d'au moins un peptide contenant de 3 à 10 résidus d'acides aminés et contenant la séquence peptidique Lysine-Proline-Valine pour prévenir, ou réduire les risques de survenue, des réactions d'hypersensibilité de contact.

10. Utilisation selon la revendication 9, dans laquelle ledit peptide est tel que défini dans l'une quelconque des revendications 2 à 7.

## Claims

1. Use of at least one peptide containing from 3 to 10 amino acid residues and containing the peptide sequence Lysine-Proline-Valine,for preparing a cosmetic composition intended to be administered by topical route, as additive intended to prevent or reduce the risks of occurrence of intolerance reactions of the skin linked to contact hypersensitivity, for users of said composition.

2. Use according to Claim 1, in which the used peptide contains from 3 to 6 amino acid residues.

3. Use according to any one of the preceding claims, in which at least one of the amino acid residues, and in particular the proline residue, of the said peptide sequence has the D configuration.

4. Use according to any one of the preceding claims, in which the amino acid residues constituting the peptide sequence Lysine-Proline-Valine all have the D configuration.

5. Use according to any one of the preceding claims, in which all the amino acid residues of the peptide have the D configuration.

6. Use according to any one of the preceding claims, in which the said peptide is a peptide in which at least one functional group is protected with a protecting group, or one of its addition salts with carboxylic organic acids, salts of alkali-metals or alkaline earth metals.

7. Use according to any one of the preceding claims, in which the said additive is the tripeptide D-Lys-D-Pro-D-Val or a derivative thereof.

8. Use according to anyone of the preceding claims, in which the said composition is chosen from lotions, creams or gels for the face, the neck or the hands, lipsticks, make-up, make-up-removing or skin cleansing compositions, mascaras, foundations, powders for the face, soaps, perfumes, nail varnishes or hair compositions such as shampoos, hair lotions, hair-dyeing compositions, permanent waving compositions for the hair.

9. Use as active ingredient, in the preparation of a medicament intended to be administered by topical route of at least one peptide containing from 3 to 10 amino acid residues and containing the peptide sequence Lysine-Proline-Valine for combating contact hypersensitivity reactions to prevent or reduce the risks of occurrence of contact hypersensitivity reactions.

10. Use according to Claim 9, in which the said peptide or peptide derivative is as defined in any one of Claims 2 to 7.

## Patentansprüche

1. Verwendung von wenigstens einem Peptid, das 3 bis 10 Aminosäurereste und die Peptidsequenz Lysin-Prolin-Valin enthält, als Zusatzstoff bei der Herstellung eines zur topischen Verabreichung bestimmten kosmetischen Präparats, um bei den Anwendern des Präparats den plötzlich auftretenden Risiken der Hautunverträglichkeitsreaktionen, die mit einer Kontakt-Überempfindlichkeit zusammenhängen, vorzubeugen oder sie zu vermindern.

2. Verwendung nach Anspruch 1, wobei das verwendete Peptid 3 bis 6 Aminosäurereste enthält.

3. Verwendung nach einem der vorhergehenden Ansprüche, wobei der wenigstens eine der Aminosäurereste, und insbesondere der Prolinrest, der Peptidsequenz die D-Konfiguration aufweist.

4. Verwendung nach einem der vorhergehenden Ansprüche, wobei die Aminosäurereste, die die Peptidsequenz Lysin-Prolin-Valin bilden, alle die D-Konfiguration aufweisen.

5. Verwendung nach einem der vorhergehenden Ansprüche, wobei alle Aminosäurereste des Peptids die D-Konfiguration aufweisen.

6. Verwendung nach einem der vorhergehenden Ansprüche, wobei das Peptid ein Peptid ist, dessen wenigstens eine funktionelle Gruppe mit einer Schutzgruppe oder mit einem von Additionssalzen mit organischen Carbonsäuren, Alkalimetallsalzen oder Erdalkalimetallsalzen geschützt ist.

7. Verwendung nach einem der vorhergehenden Ansprüche, wobei der Zusatzstoff das Tripeptid D-Lys-D-Pro-D-Val ist.

8. Verwendung nach einem der vorhergehenden Ansprüche, wobei das Präparat aus Lotionen, Cremes oder Gels für Gesicht, Hals oder Hände, Lippenstiften, Makeup-Präparaten, Abschmink-Präparaten oder Hautreinigungs-Präparaten, Mascaras, Fond de Teints, Gesichtspudern, Seifen, Parfüms, Nagellacken, Shampoons, Haarlotionen, Haarfarbe-Präparaten und Dauerwell-Präparaten ausgewählt ist.

9. Verwendung als Wirkstoff bei der Herstellung eines zur topischen Verabreichung bestimmten Medikaments aus wenigstens einem Peptid, das 3 bis 10 Aminosäurereste und die Peptidsequenz Lysin-Prolin-Valin enthält, um den plötzlich auftretenden Risiken der Kontaktüberempfindlichkeitsreaktionen vorzubeugen oder sie zu vermindern.

10. Verwendung nach Anspruch 9, wobei das Peptid wie in einem der Ansprüche 2 bis 7 definiert ist.
